(19) Europäisches Patentamt
European Patent Office
Office européen des brevets

(11) Publication number: **0 439 555 B1**

(12) # EUROPEAN PATENT SPECIFICATION

(45) Date of publication of patent specification: **08.03.95**

(51) Int. Cl.[6]: **C08B 37/08**, C08B 37/10, A61K 31/725

(21) Application number: **90900186.9**

(22) Date of filing: **13.10.89**

(86) International application number: **PCT/EP89/01214**

(87) International publication number: **WO 90/04607 (03.05.90 90/10)**

(54) **OLIGOSACCHARIDES HAVING ANTIATHEROSCLEROTIC ACTIVITY.**

(30) Priority: **21.10.88 IT 2238688**

(43) Date of publication of application: **07.08.91 Bulletin 91/32**

(45) Publication of the grant of the patent: **08.03.95 Bulletin 95/10**

(84) Designated Contracting States: **AT BE CH DE FR GB IT LI LU NL SE**

(56) References cited:
**WO-A-60/6729**

**Carbohydrate Research, vol. 80, 1980, Lars-Ake Fransson et al.: "Periodate oxidation and alkaline degradation of heparinrelated glycans", see abstract, page 131 and 134-138**

(73) Proprietor: **OPOCRIN S.p.A. LABORATORIO FARMACOBIOLOGICO**
**Via Pacinotti 3**
**I-41040 Corlo (Modena) (IT)**

(72) Inventor: **MASCELLANI, Giuseppe**
**Via Pacinotti, 3**
**I-41040 Corlo (IT)**
Inventor: **BIANCHINI, Pietro**
**Via Pacinotti, 3**
**I-41040 Corlo (IT)**

(74) Representative: **Bianchetti, Giuseppe**
**Studio Consulenza Brevettuale,**
**Via Rossini, 8**
**I-20122 Milan (IT)**

EP 0 439 555 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The various pharmacological activities of heparin, heparan sulfate, dermatan sulfate are almost always related to the presence of characteristic structural blocks which are contained in the longest polysaccharide chain. As an example, the more important requirement for the anticoagulant activity of heparin consists in the presence of a specific pentasaccharide containing a D-glucuronic acid residue and only one trisulfated D-glucosamine residue (Thunberg L. et al.,Carbohydr. Res., 100, 393, 1982; Casu B. et al., Bioch. J., 197, 599,1981; Choay J., Biochem. Biophys. Res. Comm., 116, 492,1983). This sequence is the real binding site for Antithrombin III (AT III). Heparin co-factor II (HC II) is activated by an oligosaccharide consisting of at least eight monosaccharides, which is contained in dermatan sulfate (Tollefsen D.M. et al., J. Biol. Chem., 262, (19), 8854,1986).

Therefore, a specific monosaccharide sequence, contained in the larger structure of the various polysaccharides, seems to be essential for the activation of specific active sites.

The present invention relates to oligosaccharides from heparin of molecular weight ranging from 2300 to 1.000 daltons. Such oligosaccharides have no anticoagulant activity neither affinity for antithrombin III (AT III), they have in vivo antiatherosclerotic activity, also by oral administration, they inhibit platelet aggregation and block spontaneous muscle contraction. The latter activity is related to the proliferation of subendothelial smooth muscle cells.

The present invention also relates to the process for the preparation of very low molecular weight heparin oligosaccharide fragments. All the heparin fractions or fragments known up to now and described in the prior art are characterized by poor anticoagulant (APTT) and antithrombotic activities related to high AT III affinity. Anyhow, all the hitherto described processes for heparin depolymerization yield fragments of lower molecular weight than those of heparin, but having different structural characteristics.

Surprisingly, the heparin oligosaccharides of the present invention retain the heparin structure, except for molecular weight, and moreover have antithrombotic activity in experimental venous thrombosis models and, surprisingly, also in the arterial ones.

A number of processes for the depolymerization of heparin have been described in the prior art; products can be obtained differing for structural characteristics peculiar to each process. Said characteristics distinguish the known compounds from the ones of the present invention.

US Patent 4,303,651 (corresponding to EP-A0 014 184) claims an oligosaccharide having 14-18 saccharide units and molecular weight of about 3.600-4.800 daltons, obtained by deaminative depolymerization with nitrous acid. The main component of said oligosaccharide is the disaccharide L-iduronosyl-2-0-sulfate-D-glucosamine-N, 6-disulfate, and unsulfated iduronic acid is present at a position distant 3-5 saccharide units from the not reducing end. The oligosaccharide contains no glucuronic acid.

US Patents 4,500,519 and 4,351,938 claim oligosaccharides obtained by depolymerizing heparin with nitrous acid: the reducing ends thereof consist of anhydromannose which can be reduced to anhydromannitol or oxidized to D-mannonic acid.

US Patents 4,401,662 and 4,474,770 claim an oligosaccharide having high antithrombotic activity, high affinity to AT III, extremely high anti-Xa activity, which oligosaccharide consists of no more than 8 monosaccharides and is obtained by depolimerization with nitrous acid or heparinase. Its structure contains glucosamine having a $OSO_3$ group also at the 3-position.

EP-A- 0 048 231 claims an oligosaccharide containing 4-8 monosaccharides, characterized by the presence of a 3-O-sulfated glucosamine and by a reducing end consisting of 2,5- anhydro-D-mannose.

The depolimerizations described in all the above cited patents involve the acetalic linkage between the amino sugar and the uronic acid. Therefore, the obtained oligosaccharides are always compounds having a whole number of disaccharides. Moreover, where the cleavage takes place, a sulfur loss always occurs.

US Patent 4,281,108 discloses a low molecular weight heparin obtained by N-desulfation through the intermediate heparamine, depolymerization by heating in acid medium, in the presence of an oxidizing agent, and subsequent resulfation. This process causes substantial changes in the heparin structures, which cannot be restored even by resulfation.

EP-A-40 144 discloses a process for alkali hydrolysis of heparin.

In EP-A-44 228 hydrolysis always occurs on alkyl or aryl esters at the heparin carboxy group, and gives oligomers of mean molecular weight of 2.000-9.000 daltons.

Both said latter processes yield oligomers having an end Δ-4,5-unsaturated uronic acid, through $\beta$-elimination.

US Patent 4,687,765 claims the pharmacological use of LMW-H of molecular weight from 2.000 to 8.000 for lysis of blood thrombi.

US Patent 4,686,288 claims a process for the preparation of mucopolysaccharides having chains consisting of more than 6 saccharide units (molecular weight >1800 daltons) and having 2,5-anhydromannose reducing ends.

The heparin oligosaccharides of the present invention are obtained by an analogous process to the one described in PCT/EP/86/00291 (WO 86/06729). Said process is based on the production of HO. free radicals, by means of divalent metals (e.g. $Cu^{++}$) and peroxides (e.g. $H_2O_2$), which are the initiators of heparin depolimerization, See scheme 1. The heparin fragments of the present invention are designed as C 2085I, having molecular weight 2300 daltons, OP 381/1/1, of molecular weight about 2.000, C2085II having mean molecular weight 1.700 and OP 381/1/2 of molecular weight 1.000. Said fragments consist of 4÷12 saccharide units and have the following structural characteristics, by which they differ from the prior art products: the end anomeric carbons are reducing and the end monosaccharides consist of N,6-disulfated glucosamine and 2-sulfated uronic acid. This is proved by the signals appearing in the 13C.NMR spectra at 92.7 and 94.4 ppm, respectively. The remaining part of the 13C.NMR spectrum shows that the heparin structure is completely retained. Gel-permeation chromatography shows a continuous pattern for the heparin oligosaccharides obtained by the present process, differing from a not continuous pattern of 600 daltons, obtained for the oligosaccharides prepared by other deaminative depolimerization processes with nitrous acid. Therefore, the chemical structure of these heparin oligosaccharides is completely different from the one claimed by the other patents.

The oligosaccharides of the present invention have lost the anticoagulant activity of heparin and have antiatherosclerotic activity and antithrombotic activity on both arterial and venous thrombosis; good bioavailability by subcutaneous and oral routes and long lasting effect.They inhibit ex vivo platelet aggregation induced by collagen, ristocetin and ADP. The parent heparin, on the contrary, induces platelet aggregation (Brace L.D., Fareed J., Seminars in Thromb. and Haemost. 11, 190, 1985; Eika C., Scand J. Haematol. 8, 248, 1972) and/or enhances platelet aggregation induced by other agents. The oligosaccharides reduce platelet adhesion. All said activities together could not be predicted on the ground of the searches carried out for EP-A-00291/86.

The pharmacological results hitherto obtained are predictive of the clinical usefulness of the oligosaccharides of the present invention, administered in oral doses of 3-15 mg/kg day, or in form of injectable doses of 1-5 mg/kg day.

The present invention also relates to all the industrial applications of the products of the invention for therapeutical uses in humans as antithrombotic, antiatherosclerotic, fibrinolytic agents having very poor or no anticoagulant activity. To this purpose, the compounds of the invention are formulated, by means of conventional techniques and excipients, in form of pharmaceutical compositions suitable for parenteral, topic and oral administrations. Examples of formulations suited to parenteral administration comprise sterile solutions in vials. Examples of formulations suited to oral administration comprise capsules, tablets and syrups, in which the active ingredient can be vehicled also in form of liposomes or micelles. Example of formulations for topical administration comprise ointments including the traditional excipients.

The following examples further illustrate the invention without limiting it.

EXAMPLE 1 (OP 381/1/1)

300 g of heparin dissolved in 2 l of water and added with 12 g of copper acetate monohydrate are placed into a reactor. 1000 ml of a 16% hydrogen peroxide are added, during 3.6 hours, to the reaction mass, keeping pH at 7.5 by means of a N sodium hydroxide solution and raising the temperature from 44 °C to 66 °C during the first reaction hour, by outer heating.

pH is adjusted to 5.5 with 120 ml of 30% acetic acid and 6 g of ascorbic acid are added. The oligosaccharides are cooled and precipitated with 3 volumes of methanol.

After filtration and drying, 192.7 g of a product are obtained, having mean molecular weight 2.900 daltons. Filtrate A is collected. 191 g of this intermediate product are dissolved in 1.300 ml of water heated to 68 °C. 400 ml of a 16% hydrogen peroxide solution are added during 2.3 hours, keeping pH at 7.5 with sodium hydroxide. After cooling, 4 volumes of methanol are added. Precipitate is recovered by filtration and dried. 120 g of a product are obtained. The whole solid material is redissolved in water and percolated on a Chelex 100 (Bio-Rad) resin column (4 cm diameter, 35 cm height). 3 Volumes of methanol are added to the percolate.

The precipitate is collected and dried. 99.51 g of a product named OP 381/1/1 are obtained.

13C.NMR spectrum of compound OP 381/1/1 shows that the heparin structure is retained with no essential changes, and it also evidences $C_1$ signals of reducing anomeric carbon atoms of iduronic acid and of the aminosugar. The compound is subjected to in vivo and in vitro biological evaluations (table I) showing

a surprising activity in arterial thrombosis (artr. thromb) and venous thrombosis (ven. thromb) experimental models, good bioavailability and a long lasting effect (up to 8 hours after the treatment, when administered subcutaneously).

The s.c. $ED_{50}$/i.v. $ED_{50}$ ratio for the oligosaccharides of the present invention is much more favorable than that of heparin. In the kaolin-induced thrombosis model in the rat (Hladovec J., Physiol. Bohemoslovaca, 24, 551-554, 1975), i.v. $ED_{50}$, s.c. $ED_{50}$ and per os $ED_{50}$ turned out to be 0.5 mg/kg, 3.6 mg/kg and 9.4 mg/kg, respectively. The ratio of said values, which is an index of product bioavailabily, is 1/7/19.

OP 381/1/1, at the concentration of 70 mcg/ml, inhibits platelet aggregation induced by 2 mcg of collagen, ristocetin-induced platelet aggregation and platelet aggregation induced by 16 mcg/ml of ADP in comparison with heparin which, under the same conditions, increases by 20% the ADP-induced aggregation. The product of the invention reduces by 49% platelet adhesion in comparison with physiological saline (in the "Adelplats" test, by Mascia-Brunelli, Hellem A.J., Scand. J. Haematol. 7, 374, 1970) causing no thrombocytopenia.

Said characteristics explain the surprising activity of OP 381/1/1 in the arterial thrombosis experimental model.

TABLE I

### Heparin oligosaccharides - Activity

| Sample | M.W. (1) | $SO_3/COO^-$ (2) | IN VITRO APTT (3) U/mg | IN VITRO AXa (4) U/mg | IN VIVO (ED50 mg/kg) Art. thromb (5) i.v. | Art. thromb (5) s.c. | Ven. thromb (6) i.v. | Ven. thromb (6) s.c. |
|---|---|---|---|---|---|---|---|---|
| C2085I | 2300 | 2.16 | 2 | 51 | 2 | 4.6 | 0.9 | 1.8 |
| OP381/1/1 | 2000 | 2.2 | 1.5 | 30 | 1.5 | 5.9 | 1.0 | 2.1 |
| C2085II | 1700 | 2.2 | 0.7 | 9 | 2.1 | 4.8 | 1.2 | 5 |
| OP381/1/2 | 1000 | | inactiv | 2.9 | 12.2 | | 4.4 | 21 |
| Heparin | 13500 | 2.22 | 170 | 172 | 1 | 8.0 | 0.2 | 4.5 |
| LMW Heparin (7) | 4500 | 2.19 | 30 | 78 | 2 | | 0.3 | |

(1) By HPLC; (2) according to Mascellani G. et al. Il farmaco Ed: pr; 43, 165, 1988; (3) according to Basu et al. N. Engl. Med. 324, 1972; (4) according to Teien A.N. et al, Thromb. Res., 8, 413, 1976; (5) according to Lavelle S.M. et al. in "Standardization of animal models of thrombosis", Proceedings Ang. Symp. Kitzbühel, H.K.Breddin 1983, 158; (6) according to Reyers et al. Thromb. Res., 18, 699, 1980; (7) product claimed in PCT EP 86/00291.

OP 381/1/2

Filtrate A, which had been collected in the first part of this example, is concentrated to 10 liters under vacuum. 3 Volumes of acetone are added: the resulting precipitate is recovered by filtration and redissolved in water (10%) and percolated in 100 ml of Chelex 100 (Bio-Rad) resin. The completely copper free percolate is freeze-dried. 15 g of OP 381/1/2 are obtained. 13C.NMR spectrum of compound OP 381/1/2

shows the typical dispersion of signals of very low molecular weight products. Moreover, very evident appear the signals assigned to the reducing anomeric carbons of the aminosugar and of uronic acids. The general heparin structure (which is particulary illustrated by the sulfamino and $C_6$-sulfate signals) has been retained.

The presence of signals assigned to $\beta$ anomeric carbons is also evident in the region of the anomeric carbons, which presence is generally masqued in heparins having higher molecular weights.

EXAMPLE 2 (C 2085)

Fractionation on gel-filtration column.

5 g of the product OP 381/1/1, obtained in example 1, are added to 50 ml of a 0.15 M NaCl solution and percolated in an Ultrogel Ac.A 202 (LKB) column (5 cm diameter, 100 cm height) at 2 ml/min. 420 ml of dead volume are discarded, and 10 ml each fractions are recovered. The first 30 fractions are discarded. Fractions 31 to 104 (740 ml) are combined (I). The 112 other fractions are separately pooled (II).

The two groups of fractions are concentrated to small volume, salted-off on Trisacryl GF 0.5 M and freeze-dried. The products C 2085 I (3.1 g) and C 2085 II (0.47 g) are obtained. The molecular weight profile is shown in fig. 1c. By way of example, the profiles of the products obtained by depolymerization with nitrous acid, according to example 1 of EP-A-14.184 (fig. 1a) and of the product obtained by depolymerization with nitrous acid according to USA Patent 4.500.519 (fig. 1b) are reported. The integers denote disaccharide units.

EXAMPLE 3 (C 2081 I)

According to a process similar to the one of example 2, but collecting only the first 81 fractions, compound C2081 I (1.04 g) is obtained.

Scheme 1 - Heparin depolymerization. Oligosaccharide structure

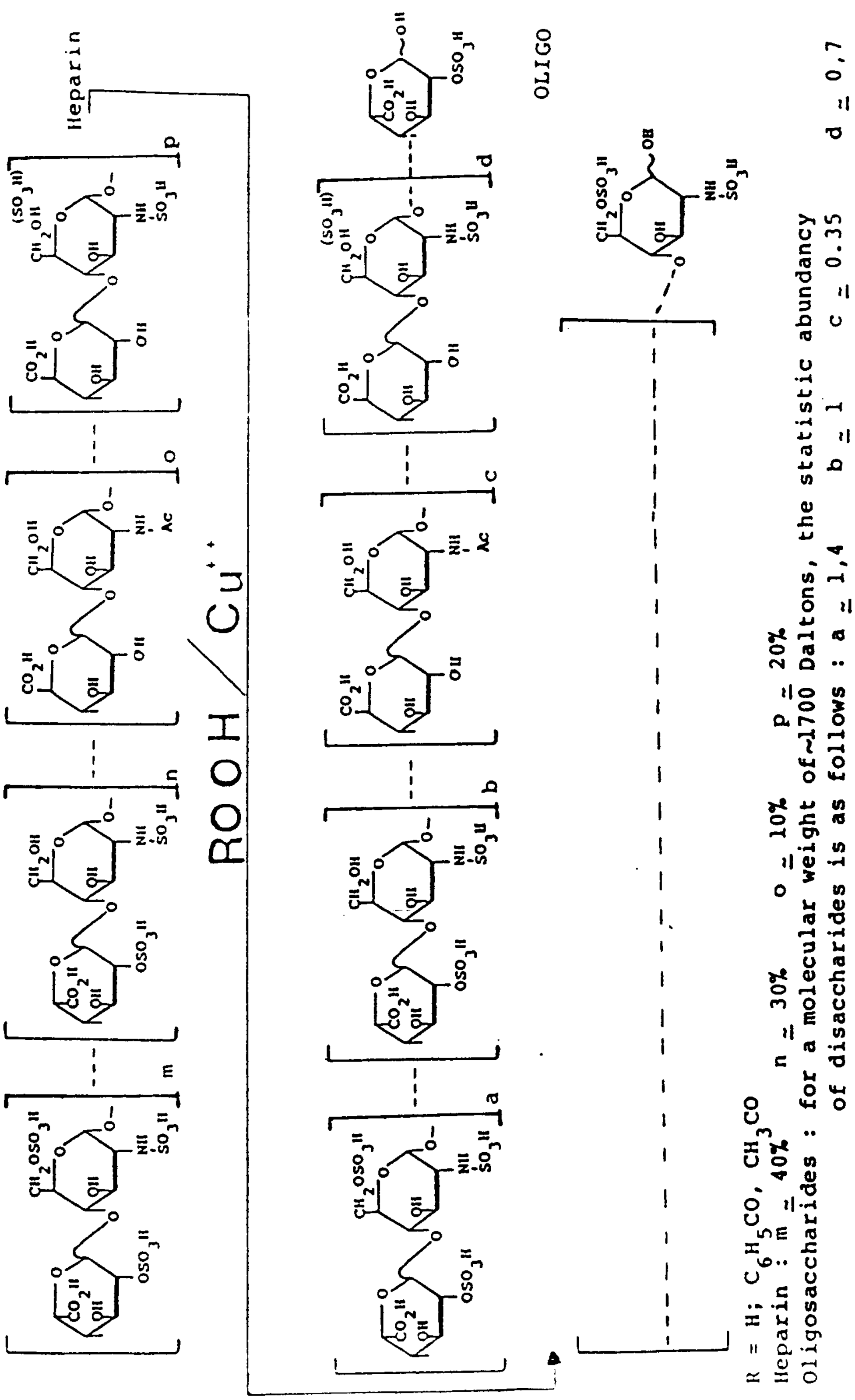


R = H; $C_6H_5CO$, $CH_3CO$

Heparin : m ≃ 40% n ≃ 30% o ≃ 10% p ≃ 20%

Oligosaccharides : for a molecular weight of ~1700 Daltons, the statistic abundancy of disaccharides is as follows : a ≃ 1,4 b ≃ 1 c ≃ 0.35 d ≃ 0,7

## Claims

1. Heparin oligosaccharide obtainable by radical depolymerizing heparin by means of divalent metals and peroxides having molecular weight lower than 3200, measured by HPLC.

2. Heparin oligosaccharide according to claim 1 having molecular weight of 2300.

3. Heparin oligosaccharide according to claim 1 having molecular weight of 2000.

4. Heparin oligosaccharide according to claim 1 having molecular weight of 1000.

5. Heparin oligosaccharide obtainable by radical depolymerising heparin by means of divalent metals and peroxides having molecular weight of 1700 measured by HPLC for use as a medicament.

6. Pharmaceutical compositions containing as the active principle an heparin oligosaccharide of claims 1-5 in combination with a suitable carrier.

**Patentansprüche**

1. Heparin-Oligosaccharid, dadurch gekennzeichnet, daß es durch radikalische Depolymerisation von Heparin mit zweiwertigen Metallen und Peroxiden hergestellt wird und ein mittels HPLC gemessenes Molekulargewicht von weniger als 3200 besitzt.

2. Heparin-Oligosaccharid nach Anspruch 1 mit einem Molekulargewicht von 2300.

3. Heparin-Oligosaccharid nach Anspruch 1 mit einem Molekulargewicht von 2000.

4. Heparin-Oligosaccharid nach Anspruch 1 mit einem Molekulargewicht von 1000.

5. Heparin-Oligosaccharid, dadurch gekennzeichnet, daß es durch radikalische Depolymerisation von Heparin mit zweiwertigen Metallen und Peroxiden hergestellt wird und ein mittels HPLC gemessenes Molekulargewicht von 1700 besitzt für die Verwendung als Arzneimittel.

6. Pharmazeutische Zubereitungen, welche als Wirkstoff ein Heparin-Oligosaccharid nach den Ansprüchen 1-6 in Verbindung mit einem geeigneten Träger enthalten.

**Revendications**

1. Oligosaccharide d'héparine pouvant être obtenu par dépolymérisation radicalaire de l'héparine au moyen de métaux divalents et de peroxydes, ayant un poids moléculaire inférieur à 3200, comme mesuré par chromatographie en phase liquide à haute performance (HPLC).

2. Oligosaccharide d'héparine selon la revendication 1, ayant un poids moléculaire de 2300.

3. Oligosaccharide d'héparine selon la revendication 1, ayant un poids moléculaire de 2000.

4. Oligosaccharide d'héparine selon la revendication 1, ayant un poids moléculaire de 1000.

5. Oligosaccharide d'héparine pouvant être obtenu par dépolymérisation radicalaire de l'héparine au moyen de métaux divalents et de peroxydes, ayant un poids moléculaire de 1700, comme mesuré par HPLC, pour une utilisation comme médicament.

6. Compositions pharmaceutiques contenant comme principe actif un oligosaccharide d'héparine des revendications 1 à 5 en combinaison avec un véhicule approprié.

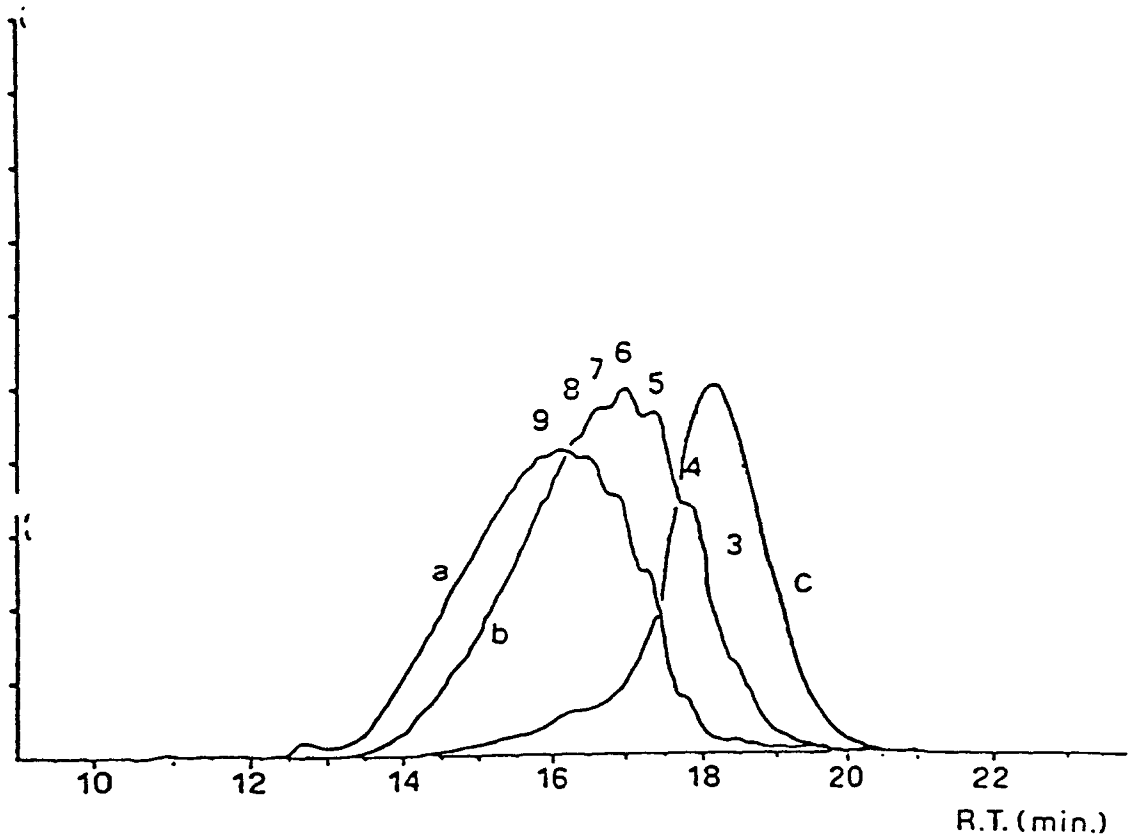


FIG. 1